# FASCICULE DE BREVET EUROPEEN

(11) **EP 0 287 488 B1**
(45) Date de publication et mention de la délivrance du brevet: **29.06.1994**
(21) Numéro de dépôt: 88440028.4
(22) Date de dépôt: 31.03.1988
(51) Int. Cl.: A61K 9/20, A61K 9/18, A61K 47/00

(54) **Médicament, produit diététique et produit d'hygiène sous la forme d'une composition pulvérulente obtenue par adsorption de principes actifs sur un sucre à dissolution rapide et procédé d'obtention de ladite composition**
Arzneimittel, diätetisches Produkt und hygienisches Produkt in Form einer pulverigen Zusammensetzung, erhalten durch Adsorption der Wirkstoffe auf einem schnellöslichen Zucker und Verfahren zur Herstellung dieser Zusammensetzung
Medicament, dietary product and hygienic product in the form of a powdery composition obtained by adsorption of the active agents on a rapidly dissolving sugar, and process for the preparation of this composition

(30) Priorité: 07.04.1987 FR 8704985
(43) Date de publication de la demande: 19.10.1988
(73) Titulaire: RECHERCHE INFORMATIQUE ET PHARMACIE R.I.PH., SARL, 67200 Strasbourg (FR)
(72) Inventeur: Jung Christophe, F-67200 Strasbourg (FR); Jung Jean, F-67200 Strasbourg (FR)
(74) Mandataire: Nuss, Pierre

(56) Documents cités:
- EP-A- 0 111 209
- EP-A- 0 196 813
- CH-A- 156 609
- DE-A- 1 667 921
- DE-A- 1 811 809
- DE-A- 3 124 574
- US-A- 4 985 252
- CHEMICAL ABSTRACTS, vol. 94, no. 18, mai 1981, page 367, résumé no. 145262n, Columbus, Ohio, US; N.M. SANGHAVI et al.: "Indomethacin-sugar dispersions", & INDIAN J. PHARM. SCI. 1980, 42(5), 136-7
- CHEMICAL ABSTRACTS, vol. 105, 1986, page 359, résumé no. 232390s, Columbus, Ohio, US; A.M. BASEDOW et al.: "Sorbitol instant an excipient with unique tableting properties", & DRUG DEV. IND. PHARM. 1986, 12(11-13), 2061-89
- Webster's Third New International Dictionary, Marriam-Webster Inc., p. 1171;
- Grand Larousse Universel, Tome 8, Larousse, Paris, p. 5614;
- The New-York Times, July 18, 1984;
- The Washington Post, August 19, 1979;
- Ullmanns Encyklopädie der technischen Chemie, 3. Aufl., 19. Bd., 1969, Urban & Schwarzenberg, München, p. 236;
- Zucker, 15 décembre 1971, no. 24, p. 753-760;

## Description

La présente invention a pour objet une composition poudreuse obtenue par adsorption de principes actifs sur un sucre à dissolution rapide, ainsi que son utilisation en tant que médicament, produit diététique et produit d'hygiène ou de soins.

On connaît actuellement des médicaments, des produits diététiques, ainsi que des produits d'hygiène sous la forme d'une composition pulvérulente obtenue par le mélange d'un ou plusieurs principes actifs avec différents excipients de nature différente, dont certains sucres du type saccharose, lactose ou glucose. Ces poudres sont de simples mélanges utilisés tels quels ou inclus dans des gélules ou des capsules, ou encore intégrés dans des formes telles que des comprimés et des suspensions.

On connaît par ailleurs du document EP-A-0196813 une composition poudreuse destinée à être dispersée sur la peau ou sur les blessures de la peau. Cette composition poudreuse est obtenue par adsorption d'un principe actif, tel que la iodine de povidone, préalablement dissout dans un solvant aqueux, sur un sucre cristallisé pulvérisé. Mais cette composition poudreuse présente, lors de son utilisation, une trop faible dispersion du principe actif dans le milieu aqueux et donc une trop faible biodisponibilité. Ceci provient en particulier du fait que la composition poudreuse présente une masse amorphe et hétérogène, c'est-à-dire que, d'une part, il subsiste des cristaux du principe actif distincts de ceux du sucre cristallisé pulvérisé et, d'autre part, chaque particule du sucre cristallisé pulvérisé ne contient le principe actif qu'à sa surface, et non à l'intérieur.

Or, le polymorphisme, la forme cristalline, la taille des particules, la vitesse de dissolution du ou des principes actifs interviennent notamment sur la biodisponibilité, en particulier lorsque le médicament est administré par voie orale et lorsque le ou les principes actifs ne sont pas totalement solubles dans l'estomac et dans le tube digestif ou lorsque la dissolution est trop lente. Il en est de même pour le produit diététique et le produit d'hygiène. En augmentant ainsi la biodisponibilité du ou des principes actifs, on augmente donc sa concentration sanguine avec une efficacité accrue du produit administré.

Le but de la présente invention est donc de réaliser des médicaments, des produits diététiques ou encore des produits hygiéniques sous forme de compositions poudreuses contenant un ou plusieurs principes actifs adsorbés à la surface extérieure et/ou intérieure d'un sucre pratiquement instantanément soluble dans l'eau, et dont la biodisponibilité du ou des principes actifs est nettement supérieure à celle de la forme classiquement utilisée en thérapeutique, en diététique, ou encore sous forme de produits d'hygiène.

Elle a, en effet, pour objet une composition poudreuse, caractérisée en ce qu'elle contient au moins une substance, dénommée principe actif, adsorbée à la surface extérieure et/ou intérieure de particules d'un sucre instantané sélectionné dans le groupe formé par le saccharose, le glucose ou le lactose, obtenue par évaporation d'un solvant organique non aqueux d'une solution organique contenant ledit principe actif ainsi que ledit sucre instantané dispersé dans celle-ci, ladite solution organique comprenant au moins un solvant organique non aqueux ne dissolvant pas ledit sucre instantané.

Le ou les principes actifs à destination médicamenteuse ou encore diététique ou encore hygiénique sont préalablement dissous dans un solvant organique non aqueux, puis sont adsorbés à la surface extérieure et/ou intérieure de particules d'un sucre instantané après évaporation de ce solvant organique non aqueux, afin d'obtenir des cristaux et/ou des particules amorphes et/ou des globules liquides de principes actifs de structure et de taille désirées.

Selon une caractéristique de l'invention, on utilisera préférentiellement comme sucre instantané du saccharose instantané. On sait, en effet, que ce produit est obtenu à la suite d'une cristallisation spéciale et présente une importante surface spécifique. De ce fait, il pourra adsorber une quantité de principes actifs plus importante, cette adsorption pouvant se faire, soit à partir d'un principe actif liquide, soit à partir d'une solution organique du principe actif. Un tel saccharose instantané pourra être celui fabriqué, par exemple, par la société Süddeutsche Zucker-Aktiengesellschaft (RFA) ou encore par la société Beghin-Say (France).

Bien entendu, d'autres sucres instantanés et composés polyhydroxylés pourront être utilisés, à condition d'être partiellement solubles dans un solvant organique non aqueux, de préférence, une solution alcoolique. Les sucres instantanés les plus couramment utilisés sont le saccharose, le glucose ou encore le lactose.

Selon une caractéristique supplémentaire de l'invention, les compositions conformes a l'invention contiennent au moins un principe actif sous la forme d'un composé ou d'un mélange de composés insolubles ou difficilement solubles dans l'eau, mais solubles dans les solvants organiques. Ces compositions pourront contenir avantageusement au moins un principe actif dépourvu d'un groupement aminé insoluble ou peu soluble dans la salive ou dans un milieu acide, tel que le suc gastrique.

Conformément à l'invention, on obtient également un ou des principes actifs sous une forme amorphe ou microcristalline, ou de globules liquides, uniforme à la surface et/ou à l' intérieur des particules de sucre instantané.

Le principe actif cristallisé examiné au microscope existe au départ sous la forme d'un mélange de particules allant d'un »m à plusieurs centaines de »m. Après adsorption sur le sucre instantané et dissolution dans l'eau, le principe actif existe, soit sous une forme amorphe et très finement dispersé dans le milieu aqueux, soit sous une forme de microcristaux de tailles voisines d'au maximum quelques »m.

La dissolution du sucre instantané au moment de l'emploi donne naissance à une très grande dispersion du ou des principes actifs préalablement adsorbés à la surface du sucre.

Les compositions objet de l'invention pourront être présentées sous une forme galénique solide sèche, en particulier sous forme de gélules, de capsules, de comprimés ou encore de suppositoires.

Dans le cas d'une gélule, celle-ci peut être ouverte au moment de l'emploi, la poudre, disposée sous la langue, favorisant ainsi le passage du ou des principes actifs dans le torrent circulatoire, le sucre instantané se dissolvant très rapidement dans la salive.

La poudre à dissolution très rapide et disposée sous la langue est particulièrement indiquée pour les traitements homéopathiques.

L'invention a également pour objet un procédé pour l'obtention d'une composition poudreuse conforme à l'invention, consistant à dissoudre au moins un principe actif dans un solvant organique non aqueux à mélanger la solution organique obtenue avec le sucre instantané, puis à faire évaporer le solvant organique non aqueux. Ce dernier pourra être, soit un éther, de préférence l'éther éthylique et homologues supérieurs, soit un ester, de préférence l'acétate d'éthyle et homologues, soit une cétone, de préférence l'acétone et homologues supérieurs, soit un acide organique non aqueux, de préférence l'acide acétique et homologues supérieurs, soit un alcool, de préférence le méthanol, l'éthanol et homologues supérieurs, soit un solvant halogéné, de préférence le chlorure de méthylène, le chloroforme et homologues halogénés, soit, enfin, un solvant hydrocarboné, de préférence l'hexane, le toluène et homologues.

Selon une variante de réalisation de l' invention, le procédé consiste à faire réagir une solution éthérée de vitamine E, principe actif liquide, avec du sucre instantané, puis à laisser évaporer le solvant jusqu'à sa complète élimination et obtention d'une masse pulvérulente.

Selon une seconde variante de réalisation de l'invention, le procédé consiste à faire réagir une ou plusieurs huiles essentielles utilisées en aromathérapie avec le sucre instantané.

Selon une troisième variante de réalisation de l'invention, le procédé consiste à faire réagir une solution de chlorure de méthylène d'indométhacine avec le sucre instantané, et à faire évaporer le solvant organique.

L'invention sera mieux comprise grâce aux exemples donnés, ci-après, à titre non limitatif :

### EXEMPLE 1 :

On dissout 2,5 g d'indométhacine dans 100 ml de chlorure de méthylène et on les mélange avec 25 g de sucre instantané fin (référence 4515) préparé par la Société Süddeutsche-Aktiengesellschaft, Werk Wagheusel. On mélange soigneusement et on fait évaporer le solvant. On homogénéise la poudre et on prépare des gélules contenant 275 mg de poudre (principe actif par gélule : 25 mg d'indométhacine).

### EXEMPLE 2 :

On dissout 3 g de vitamine E dans 100 ml d'éther éthylique et on les mélange avec 20 g de sucre instantané (référence 4515) préparé par la Société Süddeutsche-Aktiengesellschaft. On mélange soigneusement et on fait évaporer le solvant. On homogénéise la poudre et on prépare des gélules contenant 230 mg de poudre (principe actif par gélulé : 30 mg de vitamine E).

### EXEMPLE 3 :

On triture 1 g de teinture-mère d'Arnica avec 9 g de sucre instantané. On prélève 1 g de cette trituration et on le mélange avec 9 g de sucre instantané. 1 g de cette deuxième dilution est trituré avec 9 g de sucre instantané ; on obtient ainsi une troisième dilution décimale à base de sucre instantané d'Arnica, préparation très rapidement dissoute après administration sublingale. Par dilutions identiques successives, on obtient ainsi les dilutions préconisées dans les traitements homéopathiques. La poudre peut être conditionnée dans des gélules qui seront ouvertes au moment de l'emploi, la poudre étant mise sous la langue.

Toute teinture-mère homéopathique peut ainsi faire l'objet de préparations homéopathiques.

### EXEMPLE 4 :

On dissout 750 mg de chacune des huiles essentielles de lavande, de sarriette, de basilic et de carvi (des huiles essentielles telles que celles préconisées dans les traitements d'affections intestinales, entérites, colites, parasitoses, voir ouvrage du Dr. J. Valnet "Aromathérapie-Traitement des maladies par les essences des plantes", Maloine Ed. 1981, page 368) dans 20 ml d'éther et on les mélange avec 18 g de sucre instantané. On mélange soigneusement et on fait évaporer le solvant. On homogénéise la poudre et on prépare des gélules contenant 300 mg de poudre.

La quantité de principe adsorbable dépend de chaque cas considéré ; cette quantité peut atteindre facilement 10 à 20 pour cent par rapport au sucre instantané et peut, dans certains cas, les dépasser.

Il va de soi que les proportions des quantités mises en oeuvre dans les exemples 1 à 5 sont transposables à une production industrielle de plusieurs centaines de kilogrammes, l'adsorption n'étant pas modifiée.

Après administration orale de 50 mg d'indométhacine (2 gélules à 25 mg), le sang est recueilli et 5 ml de plasma sont extraits par trois fois 5 ml d'éther. Après évaporation du solvant, le résidu est repris par 0,1 ml d'éthanol. Après séparation par chromatographie en couche mince et détermination quantitative par densitométrie, on constate que la biodisponibilité est meilleure pour l'indométhacine adsorbé sur le sucre instantané, en l'occurrence 2,5 ± 0,36 »g/ml, une heure après l'administration, pour l'indométhacine adsorbé sur sucre instantané pour 1,35 ± 0,31 »g/ml pour la forme gélule utilisée en thérapeutique ou 2,2 ± 0,37 »g/ml, deux heures après l'administration, pour l'indométhacine adsorbé sur sucre instantané pour 1,3 ± 0,54 »g/ml pour la forme gélule utilisée en thérapeutique.

## Revendications

1. Composition poudreuse, caractérisée en ce qu'elle contient au moins une substance, dénommée principe actif, adsorbée à la surface extérieure et/ou intérieure de particules d'un sucre instantané sélectionné dans le groupe formé par le saccharose, le glucose ou le lactose, obtenue par évaporation d'un solvant organique non aqueux d'une solution organique contenant ledit principe actif ainsi que ledit sucre instantané dispersé dans celle-ci, ladite solution organique comprenant au moins un solvant organique non aqueux ne dissolvant pas ledit sucre instantané.

2. Composition, suivant la revendication 1, caractérisée en ce que le principe actif est sous la forme d'un composé ou d'un mélange de composés insolubles ou difficilement solubles dans l'eau, mais solubles dans les solvants organiques.

3. Composition, suivant l'une quelconque des revendications 1 et 2, caractérisée en ce qu'elle contient avantageusement au moins un principe actif dépourvu d'un groupement aminé insoluble ou peu soluble dans la salive ou dans un milieu acide, tel que le suc gastrique.

4. Composition, suivant l'une quelconque des revendications 1 à 3, caractérisée en ce qu'au moins un principe actif se trouve sous une forme amorphe ou microcristalline ou de globules liquides, uniforme à la surface et/ou à l'intérieur des particules de sucre instantané.

5. Composition, suivant la revendication 1, pour utilisation comme médicament.

6. Composition, suivant la revendication 1, pour utilisation comme produit diététique.

7. Composition, suivant la revendication 1, pour utilisation comme produit d'hygiène ou de soins.

8. Composition, suivant l'une quelconque des revendications 5 à 7, caractérisée en ce qu'elle est présentée sous une forme galénique solide sèche, en particulier sous forme de gélules, de capsules, de comprimés ou encore de suppositoires.

9. Composition, suivant l'une quelconque des revendications 5 à 8, caractérisée en ce que la dissolution dans l'eau du sucre instantané au moment de l'emploi donne naissance à une très grande dispersion du ou des principes actifs, ces derniers continuant à exister après dissolution dans l'eau soit sous une forme amorphe et très finement dispersés, soit sous une forme de microcristaux de tailles voisines d'au maximum quelques microns, ce qui augmente la biodisponibilité et donc leur concentration sanguine et ainsi l'efficacité du produit administré.

10. Composition, suivant l'une quelconque des revendications 5 à 9, caractérisée en ce que dans le cas d'une gélule, celle-ci peut être ouverte au moment de l'emploi, la poudre, disposée sous la langue, favorisant ainsi le passage du ou des principes actifs dans le torrent circulatoire, le sucre instantané se dissolvant très rapidement dans la salive.

11. Composition, suivant la revendication 10, caractérisée en ce que la poudre à dissolution très rapide et disposée sous la langue est particulièrement indiquée pour les traitements homéopathiques.

12. Procédé d'obtention d'une composition poudreuse, suivant l'une quelconque des revendications 1 à 11, caractérisé en ce qu'il consiste à dissoudre au moins un principe actif dans un solvant organique non aqueux, à mélanger la solution organique obtenue avec le sucre instantané, puis à faire évaporer le solvant organique non aqueux.

13. Procédé, suivant la revendication 12, caractérisé en ce qu'il consiste à faire réagir une solution éthérée de vitamine E, principe actif liquide, avec du sucre instantané, puis à laisser évaporer le solvant jusqu'à complète élimination et obtention d'une masse pulvérulente.

14. Procédé, suivant la revendication 12, caractérisé en ce qu'il consiste à faire réagir une ou plusieurs huiles essentielles utilisées en aromathérapie avec le sucre instantané.

15. Procédé, suivant la revendication 12, caractérisé en ce qu'il consiste à faire réagir une solution de chlorure de méthylène d'indométacine avec le sucre instantané, et à faire évaporer le solvant organique.

16. Procédé, suivant l'une quelconque des revendications 12 à 15, caractérisé en ce qu'on utilise comme solvant organique, soit un éther, de préférence l'éther éthylique et homologues supérieurs, soit un ester, de préférence l'acétate d'éthyle et homologues, soit une cétone, de préférence l'acétone et homologues supérieurs, soit un alcool pur, de préférence le méthanol, l'éthanol et homologues supérieurs, soit un solvant halogéné, de préférence le chlorure de méthylène, le chloroforme et homologues halogénés, soit, enfin, un solvant hydrocarboné, de préférence l'hexane, le toluène et homologues.

## Claims

1. Powder composition, characterised in that it contains at least one substance designated as active ingredient, adsorbed on the external and/or internal surface of particles of an instantaneous sugar selected from the group formed by saccharose, glucose or lactose and obtained by evaporation of a non-aqueous organic solvent from an organic solution containing said active ingredient as well as said instantaneous sugar dispersed therein, said organic solution comprising at least one non-aqueous organic solvent which does not dissolve said instantaneous sugar.

2. Composition according to Claim 1, characterised in that the active ingredient is in the form of a compound or a mixture of compounds which are insoluble or difficultly soluble in water but soluble in organic solvents.

3. Composition according to any one of Claims 1 or 2, characterised in that it advantageously contains at least one active ingredient which is free from an amine group which is insoluble or sparingly soluble in saliva or in an acidic medium such as gastric juice.

4. Composition according to any one of Claims 1 to 3, characterised in that at least one active ingredient is in an amorphous or microcrystalline form or in the form of liquid globules and is uniform at the surface and/or in the interior of the instantaneous sugar particles.

5. Composition according to Claim 1 for use as a medicament.

6. Composition according to Claim 1, for use as a dietetic product.

7. Composition according to Claim 1 for use as a hygiene or health-care product.

8. Composition according to any one of Claims 5 to 7, characterised in that it is presented in a dry solid galenical form, in particular in the form of soft and hard capsules, tablets or as suppositories.

9. Composition according to any one of Claims 5 to 8, characterised in that the dissolution in water of the instantaneous sugar at the moment of use gives rise to very great dispersion of the active ingredient or ingredients, these active ingredients continuing to exist after dissolution in water either in an amorphous, very finely dispersed form or in the form of microcrystals having maximum sizes of about a few microns, increasing their biocompatibility and therefore their concentration in the blood and hence the effectiveness of the product administered.

10. Composition according to any one of Claims 5 to 9, characterised in that in the case of a soft capsule, the soft capsule may be opened at the moment of use, the powder placed beneath the tongue thus promoting the passage of the active ingredient or ingredients into the circulation, the instantaneous sugar dissolving very rapidly in saliva.

11. Composition according to Claim 10, characterised in that the very rapidly dissolving powder placed beneath the tongue is particularly indicated for homeopathic treatments.

12. Process for obtaining a powder composition according to any one of Claims 1 to 11, characterised in that it involves dissolving at least one active ingredient in a non-aqueous organic solvent, mixing the organic solution obtained with the instantaneous sugar then evaporating the non-aqueous organic solvent.

13. Process according to Claim 12, characterised in that it involves reacting an ethereal solution of Vitamin E, the liquid active ingredient, with instantaneous sugar then allowing the solvent to evaporate until it has been completely eliminated and a powdered mass is obtained.

14. Process according to Claim 12, characterised in that it involves reacting one or more essential oils used in aromatherapy with the instantaneous sugar.

15. Process according to Claim 12, characterised in that it involves reacting a solution of indomethacin methylene chloride with the instantaneous sugar and evaporating the organic solvent.

16. Process according to any one of Claims 12 to 15, characterised in that the organic solvent used is an ether, preferably ethyl ether and higher homologues, or an ester, preferably ethyl acetate and homologues, or a ketone, preferably acetone and higher homologues, or a pure alcohol, preferably methanol, ethanol and higher homologues or a halogenated solvent, preferably methylene chloride, chloroform and halogenated homologues or finally a hydrocarbonated solvent, preferably hexane, toluene and homologues.

## Patentansprüche

1. Pulverige Zusammensetzung, dadurch gekennzeichnet, daß sie wenigstens einen Stoff, Wirkstoff genannt, enthält, der auf der äußeren und/oder auf der inneren Oberfläche von Teilchen eines schnellöslichen Zuckers adsorbiert ist, wobei es sich bei dem Zucker um Saccharose, Glukose oder Lactose handelt, erhalten durch Verdampfen eines organischen nichtwässrigen Lösungsmittels einer organischen Lösung, die den Wirkstoff sowie den in ihr dispergierten schnellöslichen Zucker enthält, wobei die organische Lösung wenigstens ein organisches nichtwässriges Lösungsmittel enthält, das den schnellöslichen Zucker nicht löst.

2. Zusammensetzung nach Anspruch 1, dadurch gekennzeichnet, daß der Wirkstoff in Form einer in Wasser nicht oder schwerlöslichen, in den organischen Lösungsmitteln aber löslichen Verbindung oder in Form eines Gemischs derartiger Verbindungen vorliegt.

3. Zusammensetzung nach Anspruch 1 oder 2, dadurch gekennzeichnet, daß sie vorteilhafterweise wenigstens einen Wirkstoff ohne Aminogruppe enthält, der in Speichel oder in einem sauren Milieu, etwa in Magensaft, unlöslich oder gering löslich ist

4. Zusammensetzung nach einem der Ansprüche 1 bis 3, dadurch gekennzeichnet, daß sich wenigstens ein Wirkstoff in amorpher oder mikrokristalliner Form oder in Form von Schmelzkügelchen gleichmäßig an der Oberfläche und/oder im Innern der Teilchen des schnellöslichen Zuckers befindet.

5. Zusammensetzung nach Anspruch 1 zur Verwendung als Arzneimittel.

6. Zusammensetzung nach Anspruch 1 zur Verwendung als diätetisches Erzeugnis.

7. Zusammensetzung nach Anspruch 1 zur Verwendung als Hygiene- oder Pflegeerzeugnis.

8. Zusammensetzung nach einem der Ansprüche 5 bis 7, dadurch gekennzeichnet, daß sie in galenischer, fester und trockener Form vorliegt, insbesondere in Form von Filmkapseln, Kapseln, Tabletten oder auch Zäpfchen.

9. Zusammensetzung nach einem der Ansprüche 5 bis 8, dadurch gekennzeichnet, daß das Lösen des schnellöslichen Zuckers in Wasser im Moment der Anwendung zu einer sehr starken Dispersion des Wirkstoff oder der Wirkstoffe führt, wobei letztere nach dem Lösen in dem Wasser fortbestehen, entweder in amorpher oder sehr fein dispergierter Form, oder in Form von Mikrokristallen mit einer Größe im Bereich von maximal einigen Mikrometern, wodurch die Bioverfügbarkeit und somit die Blutkonzentration der Wirkstoffe und somit die Wirksamkeit des eingenommenen Erzeugnisses erhöht wird.

10. Zusammensetzung nach einem der Ansprüche 5 bis 9, dadurch gekennzeichnet, daß im Falle einer Filmkapsel diese im Moment der Anwendung geöffnet werden kann und wobei, wenn das Pulver unter der Zunge angeordnet ist, der Übergang des Wirkstoffs oder der Wirkstoffe in den Blutkreislauf dadurch begünstigt wird, daß sich der schnellösliche Zucker sehr schnell im Speichel löst.

11. Zusammensetzung nach Anspruch 10, dadurch gekennzeichnet, daß das sehr schnell lösliche und unter der Zunge angeordnete Pulver insbesondere für homöopathische Behandlungen indiziert ist.

12. Verfahren zur Herstellung einer pulverigen Zusammensetzung nach einem der Ansprüche 1 bis 11, dadurch gekennzeichnet, daß es darin besteht, wenigstens einen Wirkstoff in einem organischen nicht wässrigen Lösungsmittel zu lösen, die erhaltene organische Lösung mit dem schnellöslichen Zucker zu mischen und anschließend das organische nichtwässrige Lösungsmittel zu verdampfen.

13. Verfahren nach Anspruch 12, dadurch gekennzeichnet, daß es darin besteht, eine ätherische Lösung von Vitamin E als flüssigen Wirkstoff mit dem schnellöslichen Zucker reagieren zu lassen und anschließend das Lösungsmittel bis zur vollständigen Abscheidung und bis zum Erhalt einer pulverigen Masse zu verdampfen.

14. Verfahren nach Anspruch 12, dadurch gekennzeichnet, daß es darin besteht, eines oder mehrere in der Aromatherapie verwendete ätherische Öle mit dem schnellöslichen Zucker reagieren zu lassen.

15. Verfahren nach Anspruch 12, dadurch gekennzeichnet, daß es darin besteht, eine Methylenchlorid-Lösung des Indometacin mit dem schnellöslichen Zucker reagieren zu lassen und das organische Lösungsmittel zu verdampfen.

16. Verfahren nach einem der Ansprüche 12 bis 15, dadurch gekennzeichnet, daß als organisches Lösungsmittel verwendet wird entweder ein Äther, vorzugsweise Äthyläther und höhere Homologe, oder ein Esther, vorzugsweise Äthylacetat und Homologe, oder ein Keton, vorzugsweise Azeton und höhere Homologe, oder ein reiner Alkohol, vorzugsweise Methanol, Äthanol und höhere Homologe, oder ein halogenisiertes Lösungsmittel, vorzugsweise Methylenchlorid, Chloroform und halogenisierte Homologe oder schließlich ein Kohlenwasserstoff-Lösungsmittel, vorzugsweise Hexan, Toluol und Homologe.
